# EUROPEAN PATENT SPECIFICATION

(11) **EP 1 262 557 B1**
(45) Date of publication and mention of the grant of the patent: **18.10.2006**
(21) Application number: 01906293.4
(22) Date of filing: 26.02.2001
(51) Int. Cl.: C12Q 1/25, C12N 5/10, C12N 15/52, G01N 33/50, G01N 33/68

(54) **PARKIN PROTEIN AS UBIQUITIN LIGASE**
DAS PARKIN-PROTEIN ALS UBIQUITINLIGASE
PROTEINE PARKINE UTILISEE COMME UBIQUITINE LIGASE

(30) Priority: 24.02.2000 JP 2000047801
(43) Date of publication of application: 04.12.2002
(73) Proprietor: Boehringer Ingelheim International GmbH, 55216 Ingelheim (DE)
(72) Inventor: MIZUNO, Yoshikuni, Tokyo 114-0014 (JP); SHIMIZU, Nobuyoshi, Sakura-shi, Chiba 285-0858 (JP)
(86) International application number: PCT/JP2001/001399
(87) International publication number: WO 2001/062957

(56) References cited:
- WO-A1-00/32787
- WO-A1-99/40191
- ABBAS ET AL: "A wide variety of mutations in the parkin gene are responsible for autosomal recessive parkinsonism in Europe" HUMAN MOLECULAR GENETICS, OXFORD UNIVERSITY PRESS, SURREY, GB, vol. 8, no. 4, April 1999 (1999-04), pages 567-574, XP002108471 ISSN: 0964-6906
- MORETT E ET AL: "A novel transactivation domain in parkin" TIBS TRENDS IN BIOCHEMICAL SCIENCES, ELSEVIER PUBLICATION, CAMBRIDGE, EN, vol. 24, no. 6, 1 June 1999 (1999-06-01), pages 229-231, XP004170196 ISSN: 0968-0004
- LORICK K L ET AL: "RING fingers mediate ubiquitin-conjugating enzyme (E2)-dependent ubiquitination" PROCEEDINGS OF THE NATIONAL ACADEMY OF SCIENCES OF USA, NATIONAL ACADEMY OF SCIENCE. WASHINGTON, US, vol. 96, September 1999 (1999-09), pages 11364-11369, XP002135694 ISSN: 0027-8424
- MOYNIHAN T P ET AL: "The Ubiquitin-conjugating enzymes UbcH7 and UbcH8 interact with RING finger/IBR motif-containing domains of HHARI and H7-AP1" JOURNAL OF BIOLOGICAL CHEMISTRY, AMERICAN SOCIETY OF BIOLOGICAL CHEMISTS, BALTIMORE, MD, US, vol. 274, no. 43, 22 October 1999 (1999-10-22), pages 30963-30968, XP002135693 ISSN: 0021-9258
- ZHANG YI ET AL: "Parkin functions as an E2-dependent ubiquitin-protein ligase and promotes the degradation of the synaptic vesicle-associated protein, CDCrel-1." PROCEEDINGS OF THE NATIONAL ACADEMY OF SCIENCES OF THE UNITED STATES, vol. 97, no. 24, 21 November 2000 (2000-11-21), pages 13354-13359, XP002232849 November 21, 2000 ISSN: 0027-8424
- KITADA T. ET AL.: 'Mutation in the parkin gene cause autosomal recessive juvenile parkinsonium' NATURE vol. 392, 1998, pages 605 - 608, XP002937879
- NOBUYOSHI SHIMIZU ET AL.: 'Parkin idenshi no cloning to sono kinou' NOU SHINKEI vol. 51, no. 6, 1999, pages 487 - 491, XP002937880
- MIZUNO Y. ET AL.: 'Genetics of parkinson's disease' BIOMED & PHARMACOTHER vol. 53, 1999, pages 109 - 116, XP002937881
- SHIMURA H. ET AL.: 'Familial parkinson disease gene product, parkin, is a ubiquitin-protein ligase' NATURE GENETICS vol. 25, no. 3, July 2000, pages 302 - 305, XP002937882

## Description

### Background of the Invention:

The present invention relates to a new use of Parkin protein (hereinafter referred to as "Parkin"). In particular, the present invention relates to a use of Parkin as ubiquitin ligase (E3). The present invention also relates to a diagnosis method of juvenile Parkinsonism on the basis of Parkin's ubiquitin ligase (E3) function. In particular, the present invention also relates to a diagnosis method of juvenile Parkinsonism by detecting a mutation of Parkin gene (hereinafter referred to as "parkin"). The present invention further relates to a method of screening medicines for treating and/or preventing juvenile Parkinsonism.

It is known that the ubiquitin pathway plays an important role in the intracellular protein decomposition which relates to the control of the amount of protein in the cells [Hershko, A et al, Annu. Rev. Biochem. 67, 425-479 (1998); and Hochstrasser, M, Annu. Rev. Genet. 30, 405-439 (1996)]. Ubiquitin is linked with a target protein through an isopeptide link of C-terminal Gly of ubiquitin and ε-NH₂ group in Lys residue of the target protein. A protein is converted into ubiquitin in the presence of three enzymatic cascade catalysts, i. e. E1 (ubiquitin activation enzyme), E2 (ubiquitin linking enzyme) and E3 (ubiquitin ligase). A polyubiquitin chain formed by the series of the reactions functions as a decomposition signal for the proteolytic attack by 26S proteasome which is an ATP-depending protease complex of eukaryotes [Coux O. et al., Annu. Rev. Biochem. 65. 801-847 (1996); and Baumeister W., et al. Cell 92, 367-380 (1998)]. Because of the variety of E2 (ubiquitin linking enzyme) and E3 (ubiquitin ligase), two or more pathways of the proteolysis in the cells are obtained [Hershko A. et al., Annu. Rev. Biochem. 67, 425-479 (1998); and Hochstrasser M., Annu. Rev. Genet. 30, 405-439 (1996)]. The details of the molecular base of E3 which most closely relates to the substrate recognition have not yet been elucidated. It is suggested at present that various RING-finger proteins relate, as E3, to the ubiquitination of proteins [Harper J. W. et al., Nature Cell Biol. 1, E5-E7 (1999); Zachariae W. et al., Genes & Dev. 13, 2039-2058 (1999): Xie Y. et al., EMBO J. 18, 6832-6844 (1999); Joazeiro C. A. P. et al., Science 286, 309-312 (1999); Lorick K. L. et al., Proc. Natl. Acad. Sci. USA 96, 11364-11369 (1999); Moynihan T. P. et al., J. Biol. Chem. 274, 30963-30968 (1999); and Martinez-Noel G. et al., FEBS Leters 454, 257-261 (1999)].

The brief description will be made on E1, E2 and E3.
E1 (ubiquitin activation enzyme) is an enzyme which first forms ubiquitin molecules and a high-energy thioester link. ATP is consumed in the formation of the high-energy thioester link with the ubiquitin molecule. E1 transfers the activated ubiquitin to the following E2 (ubiquitin linking enzyme) .
E2 receives the activated ubiquitin from E1 to form a new thioester intermediate. It was already found that many of E2 selectively interact with E3 (ubiquitin ligase), some E2 directly ubiquitinate substrate proteins. E2 forms a gene family. 13 genes were identified in budded yeast. It is now being elucidated that in higher animals such as human beings, a larger number of genes are present.
E3 (ubiquitin ligase) directly or indirectly interact with a substrate protein (target protein) to catalyze a reaction of transferring ubiquitin to the substrate or a ubiquitin chain already formed in the substrate. E3 is the most important functional molecule for the determination of the substrate specificity of the ubiquitin system. Number of E3 enzymes reported until now is only small, and the functions of them have been scarcely elucidated. At present, E3 enzymes are classified into two groups of HECT type and RING finger type. The relationship between abnormality of E3 enzymes and diseases has been suggested.

(1) HECT type: a family of E3 enzymes having HECT (homologous to E6-AP at C-terminus) domain homologous to E6-AP (E6-associated protein) at C-terminus; and
(2) RING finger type: The following 6 RING finger types have already been known:
   (2-1) E-3α (a homologue of UBR1 of yeast) and E3β which recognize N-terminal of substrate (target)
   (2-2) Component APC11 of APC (anaphase promoting complex) having a molecular weight of about 1,500 KDa effective mainly in a cell division period,
   (2-3) ROC1 which is a linking factor of SCF [SkpI/Cullin 1 (Cdc53)/F-box protein] complex effective mainly in G1 period,
   (2-4) MDM2 which relates to the ubiquitination of p53,
   (2-5) Adapter protein c-Cb1 of receptor tyrosine kinase, and
   (2-6) Mammary cancer-causing gene product BRCA1.

Recently, some genes causative hereditary nerve degeneration diseases were identified, and the dynamics of abnormal proteins produced by the causative genes is now being elucidated. Consequently, it was found that many abnormal proteins aggregate and deposit in the brains and nerves which are damaged by the diseases. Therefore, a common molecular mechanisms caused by the deposition of the abnormal proteins are assumed in the nerve degeneration. It is now tried to develop a new treatment method by elucidating the mechanisms.

Parkinson's disease is a typical nerve degeneration disease mainly characterized by extrapyramidal symptoms. Pathological characteristics of Parkinson's disease are that nigra dopamigenergic neurons and other brain stem neurons are degenerated and fall out and that Lewy bodies appear in the cell bodies of remaining neurons. It was found recently that α-synuclein protein and ubiquitin are accumulated in the Lewy bodies of neurons of patients. The missense variation of a-synuclein genes was also found, and it was suggested that this variation is a cause of the abnormal intracellular accumulation.

Recently, genes causing autosomal recessive juvenile Parkinson's disease (AR-JP) of young human beings, which is of recessive inheritance, were identified after the linkage analysis, and they were named "parkin" [Kitada T. et al., Nature 392, 605-608 (1998)]. "parkin" was a new gene derived from human No. 6 chromosome q 25.2-27. The protein (Parkin) encoded with parkin has a domain [ubiquitin-like (Ubl) domain] homologous to the ubiquitin at the N-terminal. Supposing Parkin concerns the proteolysis like ubiquitin, losing of the function thereof causes accumulation of some proteins (abnormal proteolysis) and injure the neurons. Parkin had a RING-box, comprising two RING finger domains and an IBR (in between RING) between them, at C-terminal. However, no evidence showing that Parkin concerns the ubiquitin pathway was reported until now. The function of Parkin in the cells has not been fully elucidated yet.

### Disclosure of the Invention:

The object of the present invention is to find a new function of Parkin by examining whether Parkin concerns the ubiquitin path or not and then to provide a novel method of diagnosing juvenile Parkinsonism on the basis of the finding. Another object of the present invention is to provide a method for screening medicines for preventing and/or treating juvenile Parkinson's disease by using Parkin having a changed function in the ubiquitin pathway.

After intensive investigations made for the purpose of solving the above-described problems, the inventors have succeeded in proving the fact that Parkin concerns the transfer reaction of ubiquitin into the target protein in the ubiquitin pathway. The present invention has been completed on the basis of this finding.

The present invention provides the use of Parkin protein (Parkin) as ubiquitin ligase (E3). In an embodiment of the present invention, ubiquitin ligase is an enzyme having an activity of linking with a ubiquitin linking enzyme (E2) to link ubiquitin with a target protein.

In still another aspect, the present invention provides a diagnosis method of juvenile Parkinsonism which comprises determining an activity of linking ubiquitin with the target protein in neurons in Parkin protein (Parkin) of the patient, and also a diagnosis method of juvenile Parkinsonism which comprises determining the interaction between Parkin protein (Parkin) of the patient with ubiquitin linking enzyme (E2).

The ubiquitin linking enzyme (E2) is preferably UbcH7 or UbcH8.

In still another aspect, the present invention provides a method of screening medicines for treating and/or preventing juvenile Parkinsonism, which comprises:
(1) a step of providing cells having an expression vector containing Parkin gene (parkin) from a patient with juvenile Parkinsonism,
(2) a step of bringing the cells into contact with a candidate compound, and
(3) a step of determining the activity of Parkin protein (Parkin) to link ubiquitin with a target protein and/or the interaction of Parkin protein (Parkin) and a ubiquitin linking enzyme (E2).

The cells are preferably those derived from nerves.

In still another aspect, the present invention provides a medicine for treating and/or preventing juvenile Parkinsonism identified by the screening method of the present invention.

### Brief Description of Drawings:

Fig. 1 is a photograph showing the results of immunoblotting, which shows the association of Parkin and UbcH7 in human fetal kidney 293 cells.
Fig. 2a is a schematic view showing the structure of Parkin and the structure of artificial or natural variant of Parkin used in Example 2. Fig. 2b is a photograph showing the results of the immunoblotting showing the association of UbcH7 with varied Parkin.
Fig. 3 is a picture showing the results of the immunoblotting, which shows the association of the ubiquitinated cell protein and Parkin after the treatment with MG132 in human dopamine neuroblastoma SH-SY5Y cell.
Fig. 4 is a picture showing the results of the immunoblotting, which shows the results of the domain analysis of Parkin necessitated for the linking with ubiquitinated protein.
Fig. 5 is a schematic view showing a model of ubiquitination pathway to which Parkin relates. In Fig. 5, Ub indicates ubiquitin, E1 indicates a ubiquitin activation enzyme, E2 represents a ubiquitin linking enzyme, Ub1 represents a ubiquitin-like domain, and "X" represents a target protein assumed to be recognized by the ubiquitin-like domain of Parkin for the ubiquitination.

### Description of the Preferred Embodiments:

The present invention relates to the use of Parkin protein (Parkin) as ubiquitin ligase (E3). The embodiment of the term "use" of Parkin as ubiquitin ligase (E3) is not particularly limited. It indicates the use of Parkin for the diagnosis of Parkinson's disease, particularly juvenile Parkinsonism, or the use thereof for screening medicines for the diagnosis, prevention and/or treatment of Parkinson's disease, particularly juvenile Parkinsonism.

Ubiquitin ligase (E3) is an enzyme directly or indirectly interact with a target protein to transfer ubiquitin into a target protein. In some cases, a ubiquitin chain has already been formed in the target protein. In such a case, ubiquitin ligase (E3) catalyzes the reaction of transferring ubiquitin into the already formed ubiquitin chain. An expression "Ubiquitin is linked with (or transferred into) a target protein" herein indicates that ubiquitin is transferred into the non-ubiquitinated target protein *per se* or it is transferred into a ubiquitin chain of already ubiquitinated target protein.

In some cases, it is necessary that ubiquitin ligase (E3) is linked with ubiquitin linking enzyme (E2) for exhibiting its effect of linking ubiquitin to the target protein.

The present invention also relates to a method of diagnosing juvenile Parkinsonism. In the diagnosis method of the present invention, a variation which exerts an influence on activity of Parkin for linking ubiquitin with the target protein in neurons and/or a variation which exerts an influence on the interaction of Parkin and the ubiquitin linking enzyme (E2).

Parkin is reported in Kitada T. et al, Nature 392, 605-608 (1998). "parkin" is composed of 12 exons and it encodes 465 amino acids. "parkin" has a ubiquitin-like domain (amino acid Nos. 1 to 76) at N-terminal, and RING1 domain (amino acid Nos. 238 to 293), IBR domains (amino acid Nos. 314 to 377) and RING2 domain (amino acid Nos. 418 to 449) at C-terminal. The parkin gene variations of Japanese and Turkish patients are analyzed. It is now being elucidated that in patients with juvenile Parkinsonism, frequency of deletion of a specified exon is high. The term "RING box" herein indicates an amino acid sequence of amino acid numbers of 238 to 449, and "around it" indicates about 10 to 20 amino acid residues around the RING box.

It is detected in Northern blotting that mRNA of Parkin is generally expressed, and the expression in the brain is particularly high. As will be shown in Examples given below, Parkin has an activity of ubiquitinating a target protein in human dopamigenergic neuroblastoma SH-SY5Y cells. The activity of Parkin to catalyze the ubiquitin linking reaction is considered to be kept by certain cells including neurons. Therefore, it is preferred to use neurons such as human dopamigenergic neuroblastoma SH-SY5Y cells.

The variation which exerts an influence on the activity of linking ubiquitin with a target protein involves a variation of either decreasing or increasing the activity of linking ubiquitin with the target protein. It is generally considered that when the linkage of ubiquitin with the target protein is reduced, the decomposition efficiency of the target protein is lowered and, as a result, the accumulation of the protein is caused, though the present invention is not limited by this theory. It is possible, therefore, that juvenile Parkinsonism can be diagnosed by determining the lowering of the ubiquitin linking activity.

The variation of Parkin which exerts an influence on the activity of linking ubiquitin with the target protein is caused by variation or lacking of a base in one or more of ubiquitin-like domain, RING1 domain, IBR domain and RING2 domain of parkin. Such a variation can be detected by directly determining the base sequence of DNA taken from a patient or it can be easily detected by PCR when the presence or absence of an already known variation is to be found.

The term "ubiquitin linking enzyme (E2)" herein indicates an enzyme which receives activated ubiquitin from E1 to form a new thioester intermediate. This enzyme is preferably UbcH7 or UbcH8. It is particularly preferably UbcH7.

UbcH7 is a protein composed of 154 amino acids and having a molecular weight of 17861Da (Ulrike Nubert et al., J. Biol. Chem., 271, 2795-2800, 1996). UbcH8 is a protein composed of 153 amino acids and having a molecular weight of 17768Da (Sushant Kumar et al., J. Biol. Chem., 272, 13548-13554, 1997).

In the ubiquitination of E6 dependent P53 derived from papilloma viruses of type 16 and type 18, E6-AP (100kDa) having hectodomain functions as a ligase. It was reported that the ubiquitin linking enzymes (E2) in this case are UbcH5, UbcH7 and UbcH8 (Sushant Kumar et al., J. Biol. Chem., 272, 13548-13554, 1997).

The homology of UbcH7 and UbcH8 is about 46 %.

In the diagnosis method of the present invention, the activity of Parkin to link ubiquitin with a target protein in neurons and/or the interaction of Parkin and ubiquitin linking enzyme (E2) is determined.

The method of determining the activity of Parkin to link ubiquitin with the target protein in neurons is not limited. For example, an expression vector containing parkin labeled with Myc or the like and a ubiquitin expression vector labeled with FLAG or the like are simultaneously transfected into neurons such as SH-SY5Y cells. The cells are divided into 2 groups. The cells in one group are treated with a proteasome inhibitor such as MG132. A certain period after the transfection, the cells are recovered. A ubiquitin-linked protein is detected by applying the immunoblotting method wherein an antibody (such as anti FLAG antibody) to the substance used for the labeling ubiquitin is used, to an immunoprecipitate prepared by using an antibody (such as antiMyc antibody) to a substance used for the labeling Parkin. When a high-molecular ubiquitinated protein is detected by this method, it is suggested that ubiquitin is lined with the target protein. When a band corresponding to the high-molecular ubiquitinated protein is not detected in the cells which were not treated with the proteasome inhibitor, it is suggested that the high-molecular ubiquitinated protein has been decomposed with the proteasome. Thus the concern of Parkin with the proteolysis can be examined.

In the diagnosis method of the present invention, the interaction between Parkin and ubiquitin linking enzyme (E2) is examined. The interaction can be determined by any method. For example, an expression vector containing parkin labeled with Myc or the like can be transfected into a proper cell together with that containing a gene encoding a ubiquitin-linked enzyme labeled with FLAG, His, HA or the like. A certain period after the transfection, a cell extract is prepared and the immunoprecipitation is conducted with an antibody (such as antiMyc antibody) against the label used for labeling Parkin. The interaction between Parkin and ubiquitin linking enzyme (E2) can be detected by the immunoblotting with the immunoprecipitate and an antibody against the label used for labeling the ubiquitin linking enzyme.

The present invention also provides a method of screening a medicine for treating and/or preventing juvenile Parkinsonism. The screening method of the present invention comprises the following steps:
(1) a step of providing cells keeping an expression vector containing parkin from a patient with juvenile Parkinsonism,
(2) a step of bringing the cells into contact with a candidate compound, and
(3) a step of determining the activity of Parkin to link ubiquitin with a target protein and/or the interaction between Parkin and ubiquitin linking enzyme (E2).

The expression vectors used in the present invention are vectors capable of expressing insertion genes preferably with animal cells, particularly preferably with human neurons. The expression vectors are preferably those capable of autonomous replication in host cells or capable of integration into chromosomes and containing a promoter at a position at which the inserted gene can be transcribed. The kinds of the expression vectors are not particularly limited and, for example, pcDNA3.1(+) vector (Invitrogen), p-Tet-On vector (Clontech), pSI Mammalian Expression Vector (Promega) and pSVK3 expression vector (Amersham Pharmacia Biotech) are usable.

The kinds of the cells usable in the present invention are not particularly limited so far as Parkin can exhibit its function as a ubiquitin ligase in the ubiquitin pathway. The cells are, however, preferably animal cells, particularly neurons and more particularly human neurons such as human dopamigenergic neuroblastoma.

When a candidate compound improves the activity of Parkin to link ubiquitin with a target protein or it improves the interaction between Parkin and ubiquitin linking enzyme (E2), the candidate compound can be selected as a candidate for a medicine for treating and/or preventing juvenile Parkinsonism. The medicine thus selected can be subjected to the subsequent tests to confirm the medicinal effect thereof.

The kinds of the candidate compounds are not particularly limited, and they include, for example, cytokines, low-molecular medicines, hormones, specificity antibodies, peptide imitations, antisense oligonucleotides and other medicines capable of altering the cell functions or protein expression.

The following Examples will further illustrate the present invention, which by no means limit the invention.

### Example

### Experiment operation:

### (1) Expression plasmid and transfection

For preparing pcDNA3.1(+)Myc and pcDNA3.1(+)FLAG vectors, oligo DNA encoding N-terminal Myc and FLAG epitopes was ligated at KpnI/BamHI domain of pcDNA3.1(+) (Invitrogen). After the amplification of wild type parkin or N-terminal-deficient variant, Ubc2(HHR6B), UbcH8 and cDNA of ubiquitin with a suitable primer, they were ligated in BamHI domain of the vector. The C-terminal-deficient variant and missense variant of parkin were prepared by introducing a variation into pcDNA3.1(+)Myc-Parkin by using a Quik Change domain specific variation inducing kit (Stratagene) according to a manual. pCAGGS-Ubc3 was already reported [Iwai K. et al., Proc. Natl. Acad. Sci. USA 96, 12436-12441 (1999)].

pCGN-Ubc4 and other pcDNA3.1(+)FLAG-Ubc were prepared from human liver cDNA library by an ordinary method. Ubc used was obtained from human beings in all the cases. Human fetal kidney 293 (HEK293) cells and human dopamigenergic neuroblastoma SH-SY5Y cells were cultured in DMEM containing 10 % bovine fetal serum, 10 µg/ml streptomycin and 100 U/ml penicillin.

The transfection was carried out by using FuGENE6 transfection reagent according to the producer (Boehringer-Mannheim)'s manual. The cells were treated with MG132 (Cbz-Leu-Leu-Leu-aldehyde) (Peptide Institute, Inc., Osaka, Japan) having a final concentration of 50 µM.

### (1) Immunological analysis

The immunoprecipitation was conducted with rabbit polyclonal antiMyc antibodies (A-14) (Santa Cruz Biotechnology) as previously reported [Suzuki H. et al., Biochem. Biophys. Res. Commun. 256, 127-132 (1999)]. The immunoblotting was conducted with biotin linked mouse monoclonal antiFLAG(M2) antibody (SIGMA), mouse monoclonal antiMyc antibody (9E10) (Santa Cruz Biotechnology), mouse monoclonal antiHA antibody (HA.11) (Berkeley Antibody Company) or mouse monoclonal antiHis antibody (RGS.His) (QIAGEN). Streptoavidin / horseradish peroxidase complex (Amersham Pharmacia Biotech) was used for detecting biotin-linked antiFLAG(M2) antibody. Example 1: Interaction between Parkin and E2 enzyme

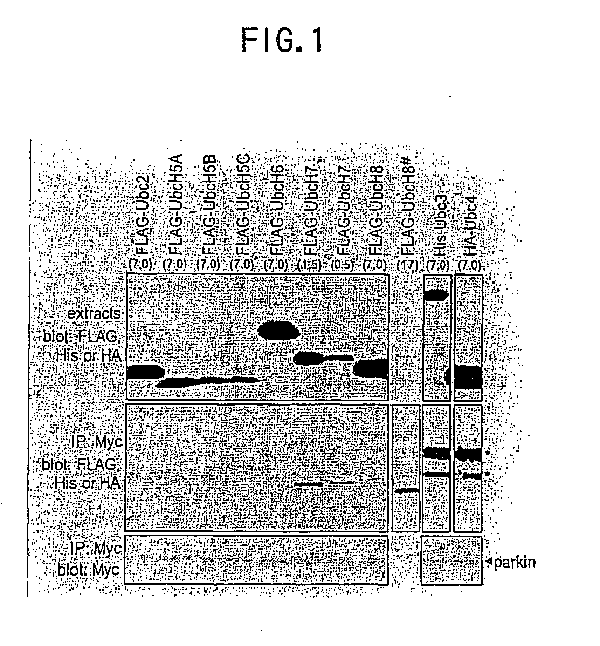

The following test was conducted to find whether Parkin concerns the ubiquitin pathway or not. To find whether the interaction between Parkin and each of various E2 enzymes occurs or not, Myc-labeled parkin was expressed together with various E2 enzymes labeled with FLAG, HA or His at different domains in HEK293 cells, and the interaction of them was detected by the immunoprecipitation The results are shown in Fig. 1.

Concretely, pcDNA3.1(+)Myc-Parkin (10 1µg) was transfected in HEK293 cells simultaneously with various expression vectors encoding FLAG-, His- or HA-Ubc in an amount shown in the parentheses in Fig. 1. 48 hours after the transfection, cell extracts were prepared, and the immunoprecipitation (IP) was conducted with antiMyc antibody. The cell extracts (upper panel in Fig. 1) and immunoprecipitates (middle and lower panels in Fig. 1) were analyzed by the immunoblotting with various antibodies as shown in Fig. 1. Although the association of Parkin and UbcH8 was not detected under ordinary conditions, it was detected when a large amount of UbcH8 was expressed and the immunoblot films were exposed for a long period of time for ECL reaction (refer to # lane). Symbol (*) indicates a nonspecific band including IgG light chain.

As shown in Fig. 1, UbcH7 and UbcH8 immunoprecipitated together with Parkin (UbcH8 immunoprecipitation was weak). However, no detectable linkage was found in other tested E2 enzymes (such as Ubc2, Ubc3, Ubc4, UbcH5A-C and UbcH6).

### Example 2

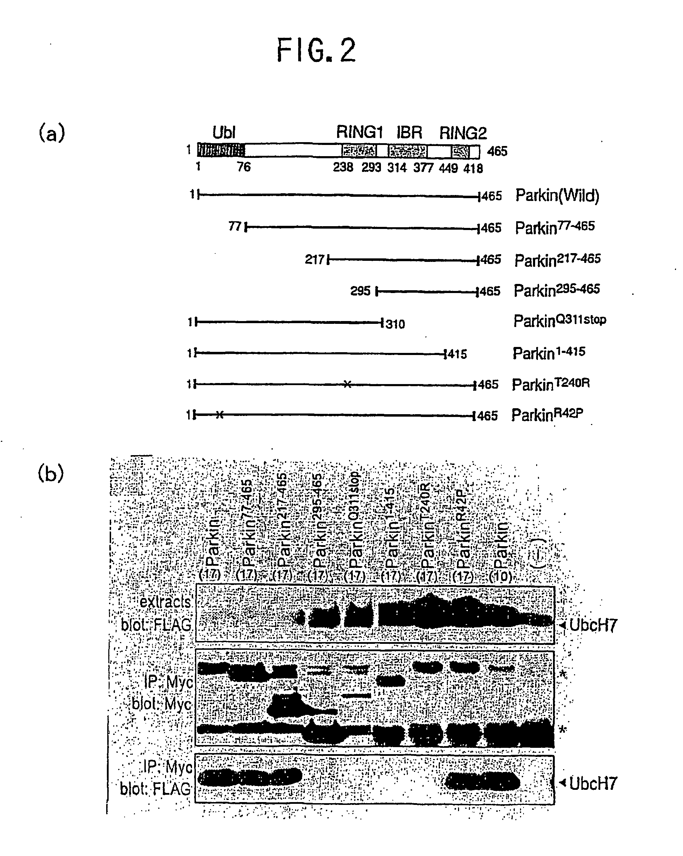

The domain structure of Parkin which can interact with UbcH7 was analyzed. Parkin has 3 domains, namely ubiquitin-like domain at N-terminal, RING-box domain at C-terminal and linker domain which links these two segments. It is known that the RING-box domain at C terminal is composed of RING1, RING2 and IBR (in-between-RING) [Morett E. et al., Trends Biochem. Sci. 24, 229-231 (1999)] as shown in Fig. 2a. Fig. 2a is a schematic view showing the structures of the artificial and natural variants of Parkin used in Example 2. Amino acid is represented by one letter notation in Fig. 2a. Nonsense isomer and missense isomer of Parkin found in patients with juvenile Parkinsonism (AR-JP) are shown as Parkin^{Q311stop} and Parkin^{T240R} / Parkin^{R42P}. Parkin^{Q311stop} is a variant formed by replacing No. 211 glutamine with stop codon, and Parkin^{T240R} / Parkin^{R42P} is a variant formed by replacing No. 240 threonine with arginine and No. 42 arginine with proline.

The interaction between variant Parkin and UbcH7 was analyzed by using the variant Parkin in the same manner as that of Example 1 except that the amount (µg) of each variant Parkin was as shown in parentheses in Fig. 2b, and pcDNA3.1(+)FLAG-UbcH7 (7 µg) was used. In the immunoblotting wherein antiFLAG antibody was used, the crude extract was used (upper panel in Fig. 2b). In the immunoblotting wherein antiMyc antibody (middle panel in Fig. 2b) or antiFLAG antibody (lower panel in Fig. 2b) was used, the immunoprecipitate obtained with antiMyc antibody was used. Symbol (*) shows the heavy chain and light chain of IgG.

It is understood from the results shown in Fig. 2b that the deletion of ubiquitin-like domain and linker domain exerted no influence on the linkage with UbcH7 and that only a half of C-terminal having RING-box was enough for the complement of UbcH7. On the contrary, RING-box variant lacking in RING1 or RING2 domain could not be linked with UbcH7. In the tests of two missense variants of patients with juvenile Parkinsonism (AR-JP), Parkin^{R42P} which was a ubiquitin-like domain variant could be linked with UbcH7, while Parkin^{T240R} in which Thr was changed to Arg [Hattori N. et al., Biochem. Biophys. Res. Commun. 249, 754-758 (1998)] did not interact with UbcH7 in RING1 domain. From these results, it was found that Ring-box domain (not other domains such as the linker domain or ubiquitin-like domain) was necessary for the association with UbcH7 which is a ubiquitin linking enzyme.

### Example 3

Tests were conducted to know whether Parkin can catch a target protein to ubiquitinate it as reported for E3 of other classes so as to elucidate the roles of Parkin in ubiquitin pathway [ZacHAriae W. et al., Genes & Dev. 13, 2039-2058 (1999); Xie Y. et al., EMBO J. 18, 6832-6844 (1999); Joazeiro, C. A. P. et al. Science 286, 309-312 (1999); and Lorick K. L. et al. Proc. Natl. Acad. Sci. USA 96, 11364-11369 (1999)].

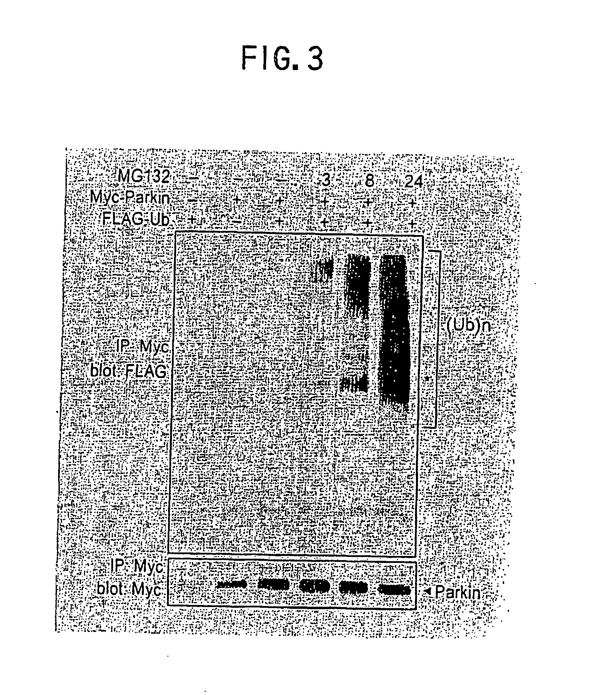

At first, 10 µg of pcDNA3.1(+)Myc-parkin and 10 µg of pcDNA3.1(+)FLAG-ubiquitin vector were simultaneously transfected into SH-SY5Y cells. 48 hours after the transfection, all the cells were recovered. In one of the experiments, the cells were treated with MG132 (50 µM) 3, 8 or 24 hours before the recovery of the cells. An immunoprecipitate prepared with antiMyc antibody was used for the immunoblotting with antiFLAG antibody (upper panel in Fig. 3) and antiMyc antibody (lower panel in Fig. 3). In Fig. 3, a symbol (*) represents the position of Parkin. A ubiquitinated protein of a high molecular weight is shown as (Ub)n on the right side.

As will be understood from the results shown in Fig. 3, when Myc-parkin and FLAG-ubiquitin were simultaneously expressed with human dopamigenergic neuroblastoma SH-SY5Y cells, ubiquitinated bands including high-molecular ubiquitinated proteins, were detected with antiMyc immunoprecipitates from a crude extract of cells which had been treated with a proteasome inhibitor MG132 (Cbz-Leu-Leu-Leu-aldehyde) [Rock K. L. et al., Cell 78, 761-771 (1994)]. On the other hand, when the treatment with an inhibitor was not conducted, no ubiquitinated band was found. The association of ubiquitinated labeled protein with Parkin proceeded depending on the time after the treatment of MG132. Some of the ubiquitinated proteins were smaller than Parkin. These results indicated that Parkin can catch the cell proteins for the ubiquitination and that it can function as ubiquitin protein ligase.

In the same analysis as above but wherein HEK293 fetal kidney cell extract was used, no ubiquitinated band linked with Parkin was observed (no data are shown). These results suggested that target protein of Parkin was lacking in HEK293 cells or another indispensable factor which exerts an influence on the linkage of Parkin with the target protein or on the E3 activity of Parkin is lacking in HEK293 cells and that ubiquitination-inhibiting protein is contained in HEK293 cells.

### Example 4

It was tried to elucidate the functional relationship between the linkage of Parkin and E2 in SH-SY5Y neurons and the ubiquitination activity.

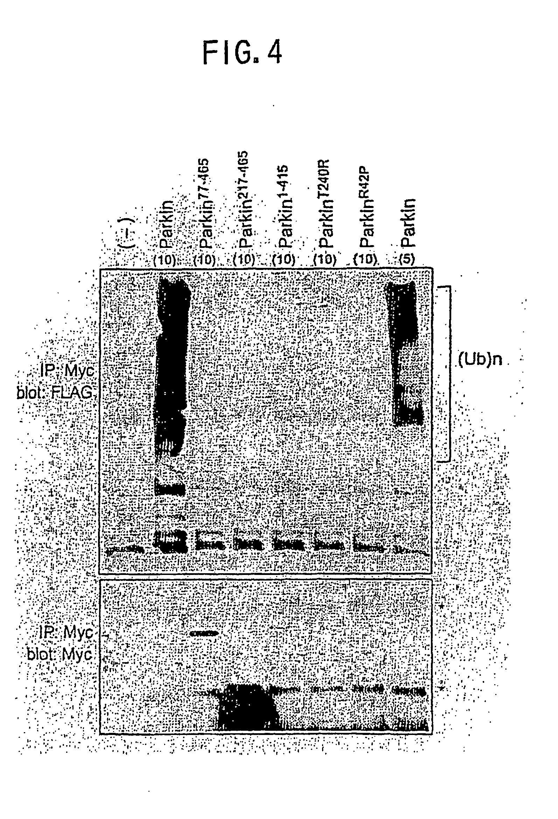

The experimental conditions were the same as those of Example 3. The expression vectors of various variants Myc-Parkin were used in an amount (µg) given in parentheses in Fig. 4. Before recovering the cells, they were treated with MG132 (50 µM) for 24 hours. 48 hours after the transfection, the immunoprecipitate prepared with antiMyc antibody was used for the immunoblotting with antiFLAG antibody (upper panel in Fig. 4) and antiMyc antibody (lower panel in Fig. 4). The details of the variant Parkin are shown in Fig. 2a. In Fig. 4, a symbol (*) represents the heavy chain and light chain bands of IgG as in Fig. 2b. The band of IgG heavy chain overlaps with the positions of wild Parkin and missense varied Parkin.

As shown in Fig. 4, when a lack or missense variation occurred in RING-box, which made the uptake of UbcH7 impossible, a band of the ubiquitinated protein associated with Parkin was not detected under the same conditions of treatment with MG132. These results indicated that the uptake of UbcH7 is indispensable for the function of Parkin. Parkin variants lacking in ubiquitin-like domain and varied Parkin^{R42P} taken from a patient substantially completely lost the association with the ubiquitinated protein (Fig. 4). However, because these varied Parkin could be linked with UbcH7 (Fig. 2b), it was suggested that the ubiquitin-like domain is indispensable for the recognition of the target. These results suggested that the loss in the ubiquitination activity of Parkin is a cause of AR-JP.

### Discussion of Examples:

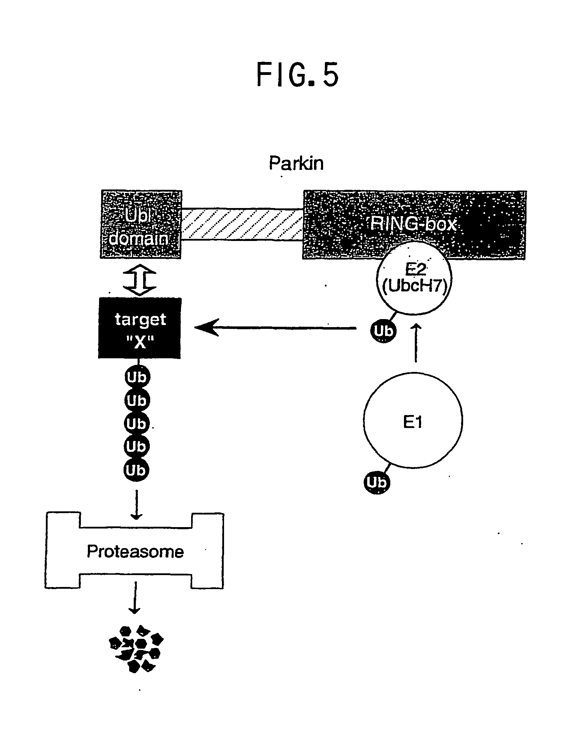

It was suggested that Parkin concerns juvenile Parkinson syndrome which causes nerve degeneration of nigra [Kitada T. et al., Nature 392, 605-608 (1998); and Mizuno Y. et al., J. Neurochem. 71, 893-902 (1998)]. The results of Examples 1 to 4 suggested that Parkin functions as a ligase (E3 enzyme) for linking ubiquitin with a target protein with UbcH7 (E2 enzyme) and that it catalyzes the ubiquitination of the target protein. Fig. 5 is a schematic view showing the mechanism. Parkin is composed of two functionally different domains, namely, (1) C-terminal RING-box for catching UbcH7 which is a specific E2 enzyme and (2) N-terminal ubiquitin-like domain necessary for the recognition of target protein for the ubiquitination. Many AR-JP patients have deficiency variation of Parkin and some of the patients have missense variation in RING-box or ubiquitin-like domain [Hattori N. et al., Biochem. Biophys. Res. Commun. 249, 754-758 (1998); and Hattori N. et al., Ann. Neurol. 44. 935-941 (1998)]. In our tests, all the Parkin constructions having a variation in this domain or lacking this domain were lacking in E3 activity. This fact indicates that the ubiquitination pathway mediated by Parkin plays an important role in keeping the homeostasis of neuron of nigra by controlling the amount of protein.

However, the mechanism of the fact that dysfunction of Parkin induces a highly selective death of neurons is still unknown. It was found in experiments according to the present invention that ubiquitinated protein is not immunoprecipitated in HEK293 cells of fetal kidneys, while it is immunoprecipitated together with Parkin in SH-SY5Y cells in nerves. This finding suggests that Parkin selectively participates in the proteolysis in neurons. When Parkin is regarded to be ubiquitin ligase, a factor indispensable for the recognition of the substrate with a target protein (shown as "X" in Fig. 5) and/or Parkin might be present in only dopamigenergic neurons. Possibly, an abnormal accumulation of "X" in the nigra cells of patients with AR-JP induces a specific death of neurons. This phenomenon can probably be explained by the finding that Parkin concerns the ubiquitination and it would act as ubiquitin protein ligase.

Evidences proving the fact that the abnormal accumulation of ubiquitinated protein is often observed in various nerve degeneration diseases are increasing more and more [Lowe J. et al., Brain Pathol. 3, 55-65 (1993); and Mayer R. et al. (1998), In "Ubiquitin and the Biology of the Cell" pp. 429-462, plenum Press, New York]. These findings indicate that the control in amount of protein intermediated by ubiquitin/proteasome pathway plays an important role in many non-dividing neurons.

Before the present invention, it was unknown that the variation of parkin in patients with AR-JP is related to the abnormality of the ubiquitination.

The function of parkin has been partially elucidated and a new diagnosis method for juvenile Parkinsonism was provided by the present invention. Also the development of a screening system for medicines usable for the prevention and/or treatment of Parkinson's disease was made possible by the present invention.

## Claims

1. The use of Parkin protein (Parkin) as a ubiquitin ligase (E3).

2. The use according to claim 1, wherein ubiquitin ligase is an enzyme having an activity of linking with a ubiquitin linking enzyme (E2) to link ubiquitin with a target protein.

3. An *in vitro* method of diagnosis of juvenile Parkinsonism which comprises determining an activity of linking ubiquitin with a target protein in neurons in Parkin protein (Parkin) of the patient.

4. An *in vitro* method of diagnosis of juvenile Parkinsonism which comprises determining the interaction between Parkin protein (Parkin) of the patient with ubiquitin linking enzyme (E2).

5. The *in vitro* method of diagnosis according to claim 4, wherein ubiquitin linking enzyme (E2) is UbcH7 or UbcH8.

6. A method of screening medicines for treating and/or preventing juvenile Parkinsonism, which comprises:
(1) a step of providing cells having an expression vector containing Parkin gene (parkin) from a patient with juvenile Parkinsonism,
(2) a step of bringing the cells into contact with a candidate compound, and
(3) a step of determining the activity of Parkin protein (Parkin) to link ubiquitin with a target protein and/or the interaction between Parkin protein (Parkin) and a ubiquitin linking enzyme (E2).

7. The screening method according to claim 6, wherein the cells are those derived from nerves.

## Patentansprüche

1. Verwendung von Parkin-Protein (Parkin) als Ubiquitin-Ligase (E3).

2. Verwendung nach Anspruch 1, wobei es sich bei der Ubiquitin-Ligase um ein Enzym mit einer Aktivität zur Verknüpfung mit einem Ubiquitin-Verknüpfungsenzym (E2) handelt, um das Ubiquitin mit einem Zielprotein zu verknüpfen.

3. In vitro-Verfahren zur Diagnose von juvenilem Parkinsonismus, das die Bestimmung einer Aktivität zur Verknüpfung von Ubiquitin mit einem Zielprotein in Neuronen im Parkin-Protein (Parkin) des Patienten umfasst.

4. In vitro-Verfahren zur Diagnose von juvenilem Parkinsonismus, das die Bestimmung der Wechselwirkung zwischen Parkin-Protein (Parkin) des Patienten mit Ubiquitin-Verknüpfungsenzym (E2) umfasst.

5. In vitro-Verfahren zur Diagnose nach Anspruch 4, wobei es sich beim Ubiquitin-Verknüpfungsenzym (E2) um UbcH7 oder UbcH8 handelt.

6. Verfahren zum Screening von Arzneimitteln für die Therapie und/oder Prophylaxe von juvenilem Parkinsonismus, umfassend:
(1) einen Schritt, bei dem Zellen mit einem Expressionsvektor, der das Parkin-Gen (Parkin) eines Patienten mit juvenilem Parkinsonismus enthält, bereitgestellt werden,
(2) einen Schritt, bei dem die Zellen in Kontakt mit einer Kandidaten-Verbindung gebracht werden, und
(3) einen Schritt, bei dem die Aktivität des Parkinson-Proteins (Parkin) zur Verknüpfung von Ubiquitin mit einem Zielprotein und/oder die Wechselwirkung zwischen dem Parkin-Protein (Parkin) und einem Ubiquitin-Verknüpfungsenzym (E2) bestimmt werden.

7. Screening-Verfahren nach Anspruch 6, wobei es sich bei den Zellen um von Nerven abgeleitete Zellen handelt.

## Revendications

1. Utilisation de la protéine Parkin (Parkin) comme ubiquitine ligase (E3).

2. Utilisation selon la revendication 1 où l'ubiquitine ligase est une enzyme ayant une activité de liaison avec une enzyme liant l'ubiquitine (E2) pour lier l'ubiquitine avec une protéine cible.

3. Procédé *in vitro* de diagnostic du parkinsonisme juvénile qui comprend la détermination d'une activité de liaison de l'ubiquitine avec une protéine cible dans des neurones dans la protéine Parkin (Parkin) du patient.

4. Procédé *in vitro* de diagnostic du parkinsonisme juvénile qui comprend la détermination de l'interaction entre la protéine Parkin (Parkin) du patient avec une enzyme liant l'ubiquitine (E2).

5. Procédé *in vitro* de diagnostic selon la revendication 4 où l'enzyme liant l'ubiquitine (E2) est UbcH7 ou UbcH8.

6. Procédé de sélection de médicaments pour traiter et/ou prévenir le parkinsonisme juvénile qui comprend :
(1) une étape de fourniture de cellules ayant un vecteur d'expression contenant un gène Parkin (parkin) d'un patient ayant un parkinsonisme juvénile,
(2) une étape de mise en contact des cellules avec un composé candidat, et
(3) une étape de détermination de l'activité de la protéine Parkin (Parkin) pour lier l'ubiquitine avec une protéine cible et/ou de l'interaction entre la protéine Parkin (Parkin) et une enzyme liant l'ubiquitine (E2).

7. Procédé de sélection selon la revendication 6 où les cellules sont celles issues de nerfs.
